(19) Europäisches Patentamt
European Patent Office
Office européen des brevets

(11) Publication number: 0 031 974 A2

# (12) EUROPEAN PATENT APPLICATION

(21) Application number: 80201175.9

(22) Date of filing: 08.12.80

(51) Int. Cl.$^3$: C 07 C 127/19
A 01 N 47/34

(30) Priority: 04.01.80 NL 8000044

(43) Date of publication of application: 15.07.81 Bulletin 81/28

(84) Designated Contracting States: AT BE CH DE FR GB IT LI NL SE

(71) Applicant: DUPHAR INTERNATIONAL RESEARCH B.V
C.J. van Houtenlaan 36
1381 CP Weesp(NL)

(72) Inventor: Brouwer, Marius Sander
c/o Octrooibureau ZOAN B.V. Apollolaan 151
NL-1077 AR Amsterdam(NL)

(72) Inventor: Grosscurt, Arnoldus Cornelis
c/o Octrooibureau ZOAN B.V. Apollolaan 151
NL-1077 AR Amsterdam(NL)

(74) Representative: Swaters, Pieter D., Drs. et al,
Octrooibureau ZOAN B.V. Apollolaan 151
NL-1077 AR Amsterdam(NL)

## (54) New urea compounds and thiourea compounds, method of preparing the new compounds, and insecticidal compositions on the basis of these compounds.

(57) The invention relates to new benzoyl ureas of the general formula

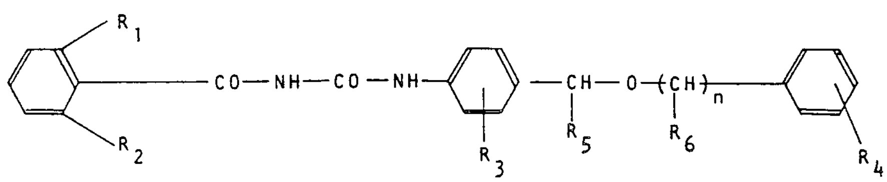

wherein $R_1$ is a hydrogen atom, a chlorine atom, a fluorine atom or a methyl group,
$R_2$ is a chlorine atom, a fluorine atom, or a methyl group,
$R_3$ is a hydrogen atom or one or two substituents selected from the group consisting of a halogen atom and an alkyl group having 1 to 4 carbon atoms,
$R_4$ is a hydrogen atom or from one to three substituents selected from the group consisting of a halogen atom and an alkyl group, alkoxy group or alkylthio group having 1 to 4 carbon atoms and substituted or not substituted with halogen,
$R_5$ and $R_6$ are equal or different and represent a hydrogen atom or an alkyl group having 1 to 4 carbon atoms and substituted or not substituted with halogen, and
$n$ is 0 or 1,
as insecticides.

After having been processed to compositions, the compounds can successfully be used for controlling insects in a dosage from 10 to 5,000 g of active substance per hectare.

EP 0 031 974 A2

DIR 0309
DUPHAR INTERNATIONAL RESEARCH B.V.

New urea compounds and thiourea compounds, method of preparing the new compounds, and insecticidal compositions on the basis of these compounds.

The invention relates to new urea compounds and thiourea compounds and to a method of preparing the new compounds. The invention also relates to insecticidal compositions on the basis of the new compounds and to the use of these compositions for the control of insects.

In Netherlands Patent Application 7105350 laid open to public inspection, benzoylurea are described having insecticidal activity. A compound mentioned in this patent application is N-(2,6-dichlorobenzoyl)-N'-[4-(4-chlorophenoxy)phenyl]-urea, of the formula below, wherein $R_1$ and $R_2$ are chlorine atoms.

These and related compounds have an interesting insecticidal activity, as appears from the results indicated below, against larvae of Pieris brassicae

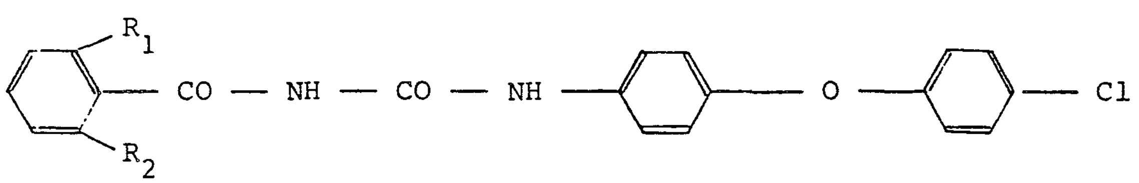

| Compound | | Conc. in mg of active substance per litre | | | | |
|---|---|---|---|---|---|---|
| $R_1$ | $R_2$ | 100 | 30 | 10 | 3 | 1 |
| Cl | Cl | + | ± | - | | |
| H | Cl | + | + | + | ± | - |

The meanings of the symbols are as follows:
+ = 90-100% mortality
± = 50-90% mortality
- = <50% mortality

It has surprisingly been found now that the corresponding 4-(4-chlorophenoxymethyl)phenylurea compound as well as related compounds have considerably stronger insecticidal properties than the already known compound. This is illustrated by the results indicated in the following survey, which results have been obtained by also determining the activities against larvae of Pieris brassicae:

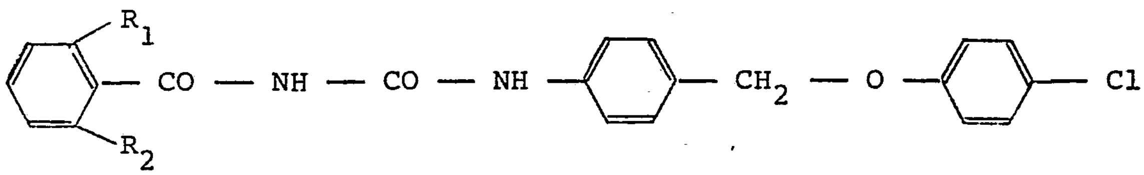

| Compound | | Conc. in mg of active substance per litre | | | | | |
|---|---|---|---|---|---|---|---|
| $R_1$ | $R_2$ | 100 | 30 | 10 | 3 | 1 | 0,3 |
| H | Cl | + | + | + | + | ± | - |
| F | F | + | + | + | + | + | + |

Therefore, the present invention is characterized by new urea compounds of the general formula

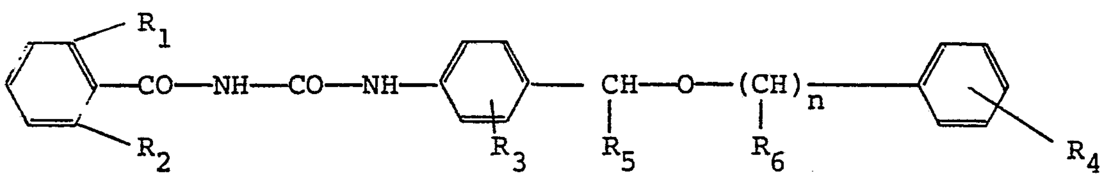

wherein $R_1$ is a hydrogen atom, a chlorine atom, a fluorine atom or a methyl group,

$R_2$ is a chlorine atom, a fluorine atom or a methyl group,

$R_3$ is a hydrogen atom or one or two substituents selected from the group consisting of a halogen atom and an alkyl group having 1 to 4 carbon atoms,

$R_4$ is a hydrogen atom or from one to three substituents selected from the group consisting of a halogen atom and an alkyl group, alkoxy group or alkylthio group having 1 to 4 carbon atoms substituted or not substituted with halogen,

$R_5$ and $R_6$ are equal or different and are a hydrogen atom or an alkyl group having 1 to 4 carbon atoms substituted or not substituted with halogen, and

n is 0 or 1.

These compounds have an interesting insecticidal activity as will become apparent from the specific examples. Of the above-mentioned compounds prove to have a very great insecticidal activity compounds of the above general formula wherein either $R_1$ and $R_2$ are both fluorine atoms, or $R_1$ is a hydrogen atom and $R_2$ is a chlorine atom.

Examples of new urea compounds having insecticidal activity are:

(1) N-(2-chlorobenzoyl)-N'-(4-phenoxymethylphenyl)urea,
(2) N-(2,6-difluorobenzoyl)-N'-(4-phenoxymethylphenyl)urea,
(3) N-(2-chlorobenzoyl)-N'-[4-(4-chlorophenoxymethyl)phenyl]urea,
(4) N-(2-methylbenzoyl)-N'-[4-(4-chlorophenoxymethyl)phenyl]urea,
(5) N-(2,6-difluorobenzoyl)-N'-[4-(4-chlorophenoxymethyl)phenyl]-urea,
(6) N-(2-chlorobenzoyl)-N'-[4-(2-methylphenoxymethyl)phenyl]urea,
(7) N-(2-methylbenzoyl)-N'-[4-(2-methylphenoxymethyl)phenyl]urea,
(8) N-(2,6-difluorobenzoyl)-N'-[4-(2-methylphenoxymethyl)phenyl]-urea,
(9) N-(2-chlorobenzoyl)-N'-[4-(2-chlorophenoxymethyl)phenyl]urea,
(10) N-(2,6-difluorobenzoyl)-N'-[4-(2-chlorophenoxymethyl)phenyl]-urea,
(11) N-(2-chlorobenzoyl)-N'-[4-(2,4-dichlorophenoxymethyl)phenyl]-urea,
(12) N-(2-methylbenzoyl)-N'-[4-(2,4-dichlorophenoxymethyl)phenyl]-urea,
(13) N-(2,6-difluorobenzoyl)-N'-[4-(2,4-dichlorophenoxymethyl)-phenyl]urea,
(14) N-(2,6-difluorobenzoyl)-N'-[4-(1-phenoxypropyl)phenyl]urea,
(15) N-(2-chlorobenzoyl)-N'-[4-(1-phenoxyethyl)phenyl]urea,
(16) N-(2-methylbenzoyl)-N'-[4-(1-phenoxyethyl)phenyl]urea,
(17) N-(2,6-difluorobenzoyl)-N'-[4-(1-phenoxyethyl)phenyl]urea,
(18) N-(2-chlorobenzoyl)-N'-[4-{1-(4-chlorophenoxy)ethyl}phenyl]-urea,

(19) N-(2-methylbenzoyl)-N'-[4-{1-(4-chlorophenoxy)ethyl}phenyl]-urea,

(20) N-(2,6-difluorobenzoyl)-N'-[4-{1-(4-chlorophenoxy)ethyl}-phenyl]urea,

(21) N-(2-chlorobenzoyl)-N'-[3-chloro-4-(phenoxymethyl)phenyl]-urea,

(22) N-(2-methylbenzoyl)-N'-[3-chloro-4-(phenoxymethyl)phenyl]-urea,

(23) N-(2,6-difluorobenzoyl)-N'-[3-chloro-4-(phenoxymethyl)-phenyl]urea,

(24) N-(2-chlorobenzoyl)-N'-[3-chloro-4-(4-chlorophenoxymethyl)-phenyl]urea,

(25) N-(2-methylbenzoyl)-N'-[3-chloro-4-(chlorophenoxymethyl)-phenyl]urea,

(26) N-(2,6-difluorobenzoyl)-N'-[3-chloro-4-(4-chlorophenoxy-methyl)phenyl]urea,

(27) N-(2-chlorobenzoyl)-N'-[4-(1-phenoxy-2,2,2-trifluoroethyl)-phenyl]urea,

(28) N-(2-chlorobenzoyl)-N'-[4-(2,6-dimethylphenoxymethyl)phenyl]-urea,

(29) N-(2-methylbenzoyl)-N'-[4-(2,6-dimethylphenoxymethyl)phenyl]-urea,

(30) N-(2,6-difluorobenzoyl)-N'-[4-(2,6-dimethylphenoxymethyl)-phenyl]urea,

(31) N-(2-chlorobenzoyl)-N'-[4-{1-(2-chlorophenoxy)ethyl}phenyl]-urea,

(32) N-(2,6-difluorobenzoyl)-N'-[4-{1-(2-chlorophenoxy)ethyl}-phenyl]urea,

(33) N-(2-chlorobenzoyl)-N'-[3-methyl-4-(phenoxymethyl)phenyl]-urea,

(34) N-(2-chlorobenzoyl)-N'-(4-benzyloxymethylphenyl)urea,

(35) N-(2-methylbenzoyl)-N'-(4-benzyloxymethylphenyl)urea,

(36) N-(2,6-difluorobenzoyl)-N'-(4-benzyloxymethylphenyl)urea,

(37) N-(2-chlorobenzoyl)-N'-[4-(1-benzyloxyethyl)phenyl]urea, and

(38) N-(2,6-difluorobenzoyl)-N'-[4-(1-benzyloxyethyl)phenyl]urea.

The substances according to the invention can be used for the control of mites and insects in agriculture and horticulture, in forests and in surface water, as well as for the protection of textile against attack by, for example, moths and carpet beetles, and against insects in stocks, for example in stored cereals.

The substances according to the invention can also be used for the control of insects living in the manure of hot-blooded animals, such as cows, pigs and hens. For this application, the active compounds can be administered orally to the animals, for example, mixed through the food, so that they land in the manure after some time ("through-feeding").

The compounds according to the invention are particularly active against larvae and eggs of insects, such as flies, mosquitoes, beetles and butterflies.

In principle, the compounds may be used against all insects mentioned in Pestic. Sci. 9, 373-386 (1978).

For practical applications the substances in accordance with the invention are usually processed to compositions. In such compositions the active substance is mixed with solid carrier material or dissolved or dispersed in liquid carrier material, if desired in combination with auxiliary substances, for example, emulsifiers, wetting agents, dispersion agents and stabilizers.

Examples of compositions according to the invention are aqueous solutions and dispersions, oily solutions and oily dispersions, solutions in organic solvents, pastes, dusting powders, dispersing powders, miscible oils, granules, pellets, invert emulsions, aerosol compositions and fumigating candles.

Dispersible powders, pastes and miscible oils are compositions in concentrate form which are diluted prior to or during use.

The invert emulsions and solutions in organic solvents are mainly used in air application, namely when large areas are treated with a comparatively small quantity of composition. The invert emulsion can be prepared shortly before or

even during spraying in the spraying apparatus by emulsifying water in an oily solution or an oily dispersion of the active substance. The solutions of the active substance in organic solvents may be provided with a phytotoxicity-reducing substance, for example, wool fat, wool fatty acid or wool fatty alcohol.

A few forms of composition will be described in greater detail hereinafter by way of example.

Granular compositions are prepared by taking up, for example, the active substance in a solvent or dispersing it in a diluent and impregnating the resulting solution/ suspension, if desired in the presence of a binder, on granular carrier material, for example porous granules (for example pumice and attaclay), mineral non-porous granules (sand or ground marlow), organic granules (for example, dried coffee grounds, cut tobacco stems and ground corncobs). A granular composition can also be prepared by compressing the active substance together with powdered minerals in the presence of lubricants and binders and disintegrating the compressed product to the desired grain size and sieving it. Granular compositions can be prepared in a different manner by mixing the active substance in powder form with powdered fillers, and glomulating the mixture then to the desired particle size.

Dusting powders can be obtained by intimately mixing the active substance with an inert solid powdered carrier material, for example, talcum.

Dispersible powders are prepared by mixing 10 to 80 parts by weight of a solid inert carrier, for example kaolin, dolomite, gypsum, chalk, bentonite, attapulgite, colloidal $SiO_2$ or mixtures of these and similar substances, with 10 to 80 parts by weight of the active substance, 1 to 5 parts by weight of a dispersing agent, for example the lignine sulphonates or alkylnaphthalene sulphonates known for this purpose, preferably also 0.5 to 5 parts by weight of a wetting agent, for example, fatty alcohol sulphates, alkyl aryl sulphonates, fatty acid condensation products, or polyoxyethylene compounds, and finally, if desired, other additives.

For the preparation of miscible oils the active compound is dissolved in a suitable solvent which preferably is poorly water-miscible, and one or more emulsifiers are added to this solution. Suitable solvents are, for example, xylene, toluene, petroleum distillates which are rich in aromates, for example, solvent naphtha, distilled tar oil and mixtures of these liquids. As emulsifiers may be used, for example, polyoxyethylene compounds and/or alkyl aryl sulphonates. The concentration of the active compound in these miscible oils is not restricted to narrow limits and may vary, for example, between 2 and 50% by weight.

In addition to a miscible oil may also be mentioned as a liquid and highly concentrated primary composition a solution of the active substance in a readily water-miscible liquid, for example, a glycol, or glycol ether, to which solution a dispersion agent and, if desired, a surface-active substance has been added. When diluting with water shortly before or during spraying, an aqueous dispersion of the active substance is then obtained.

An aerosol composition according to the invention is obtained in the usual manner by incorporating the active substance, if desired in a solvent, in a volatile liquid to be used as a propellant, for example, a mixture of chlorine-fluorine derivatives of methane and ethane, a mixture of lower hydrocarbons, dimethyl ether, or gases such as carbon dioxide, nitrogen and nitrous oxide.

Fumigating candles or fumigating powders, i.e. compositions which, while burning, can generate a pesticidal smoke, are obtained by taking up the active substance in a combustible mixture which may contain as a fuel a sugar or a wood, preferably in a ground form, a substance to maintain combustion, for example, ammonium nitrate or potassium chlorate, and furthermore a substance to delay combustion, for example, kaolin, bentonite and/or colloidal silicic acid.

In addition to the above-mentioned ingredients, the agents according to the invention may also contain other substances known for use in this type of agents.

For example, a lubricant, for example, calcium stearate or magnesium stearate, may be added to a dispersible powder or a

mixture to be granulated. "Adhesives", for example, polyvinylalcohol cellulose derivatives or other colloidal materials, such as casein, may also be added so as to improve the adhesion of the presticide to the crop. Furthermore, a substance may be added to reduce the phytotoxicity of the active substance, carrier material or auxiliary substance, for example, wool fat or wool fatty alcohol.

Pesticidal compounds known per se may also be incorporated in the compositions according to the invention. As a result of this the activity spectrum of the composition is widened and synergism may occur.

For use in such a combination composition are to be considered the following known insecticidal, acaricidal and fungicidal compounds.

Insecticides, for example:

1. organic chlorine compounds, for example 6,7,8,9,10,10-hexachloro-1,5,5a,6,9,9a-hexahydro-6,9-methano-2,4,3-benzo[e]-dioxathiepine-3-oxide;
2. carbamates, for example, 2-dimethylamino-5,6-dimethylpyrimidin-4-yl dimethyl carbamate and 2-isopropoxyphenyl methylcarbamate;
3. di(m)ethylphosphates, for example, 2-chloro-2-diethylcarbamoyl-1-methylvinyl—, 2-methoxycarbonyl-1-methylvinyl—, 2-chloro-1-(2,4-dichlorophenyl)vinyl—, and 2-chloro-1-(2,4,5-trichlorophenyl)vinyl di(m)ethyl phosphate;
4. 0,0-di(m)ethyl phosphorothioates, for example, 0(S)-2-methylthioethyl—, S-2-ethylsulphinylethyl—, S-2-(1-methylcarbamoylethylthio)ethyl—, 0-4-bromo-2,5-dichlorophenyl—, 0-3,5,6-trichloro-2-pyridyl—, 0-2-isopropyl-5-methylpyrimidin-4-yl—, and 0-4-nitrophenyl 0,0-di(m)ethyl phosphorothioate;
5. 0,0-di(m)ethyl phosphorodithioates, for example, S-methylcarbamoylmethyl—, S-2-ethylthioethyl—, S-(3,4-dihydro-4-oxobenzo[d]-1,2,3-triazin-3-ylmethyl)—, S-1,2-di(ethoxycarbonyl)ethyl—, S-6-chloro-2-oxobenzoxazolin-3-ylmethyl—, and S-2,3-dihydro-5-methoxy-2-oxo-1,3,4-thiadiazol-3-ylmethyl 0,0-di(m)ethylphosphorodithioate;

6. phosphonates, for example, dimethyl 2,2,2-trichloro-1-hydroxyethylphosphonate;
7. natural and synthetic pyrethroids;
8. amidines, for example, N'-(2-methyl-4-chlorophenyl)-N,N-dimethylformamidine; and
9. microbial insecticides, such as Bacillus thuringiensis.

Acaricides, for example:

1. organic tin compounds, for example, tricyclohexyl tin hydroxide and di[tri-(2-methyl-2-phenylpropyl)tin]oxide;
2. organic halogen compounds, for example isopropyl 4,4'-dibromobenzilate, 2,2,2-trichloro-1,1-di(4-chlorophenyl)ethanol and 2,4,5,4'-tetrachlorodiphenyl sulphone;

and furthermore: 3-chloro-α-ethoxyimino-2,6-dimethoxybenzyl benzoate and O,O-dimethyl S-methylcarbamoyl methyl phosphorothioate.

Fungicides, for example:

1. organic tin compounds, for example, triphenyl tin hydroxide and triphenyl tin acetate;
2. alkylene bisdithiocarbamates, for example, zinc ethylenebisdithiocarbamate and manganese ethylene bisdithiocarbamate;
3. 1-acyl- or 1-carbamoyl-N-benzimidazole (-2) carbamates and 1,2-bis (3-alkoxycarbonyl-2-thiureido)benzene, and furthermore

2,4-dinitro-6-(2-octylphenylcrotonate), 1-[bis(dimethylamino) phosphoryl]-3-phenyl-5-amino-1,2,4-triazole, N-trichloromethylthiophthalimide, N-trichloromethylthiotetrahydrophthalimide, N-(1,1,2,2-tetrachloroethylthio)-tetrahydrophthalimide, N-dichlorofluoromethylthio-N-phenyl-N,N'-dimethylsulphamide, tetrachloroisophthalonitrile, 2-(4'-thiazolyl)-benzimidazole, 5-butyl-2-ethylamino-6-methylpyrimidine-4-yl-dimethylsulphamate, 1-(4-chlorophenoxy)-3,3-dimethyl-1(1,2,4-triazole-1-yl)-2-butanone, α-(2-chlorophenyl)-α-(4-chlorophenyl)-5-pyrimidinemethanol, 1-(isopropylcarbamoyl)-3-(3,5-dichlorophenyl)hydantoin, N-(1,1,2,2-tetrachloroethylthio)-4-cyclohexene-1,2-carboximidine, N-trichloromethylmercapto-4-cyclohexene-1,2-dicarboximidine, N-tridecyl-2,6-dimethylmorpholine, and 5,6-dihydro-2-methyl-1,4-oxathiine-3-carboxanilide. The dosages of the composition according to the invention desired for practical application will, of course, depend on various factors, for example, field of application,

selected active substance, form of composition, nature and extent of the infection and the weather conditions.
In general it holds that favourable results are achieved with a dosage which corresponds to 10-5000 g of the active substance per hectare.

For the above-described "through-feeding", the active substance is mixed through the food in a quantity which is effective for insecticidal application.

The compounds according to the invention are new substances which can be prepared in a manner known per se for related compounds.

For example, the compounds according to the invention can be prepared by reacting a substituted aniline of the general formula

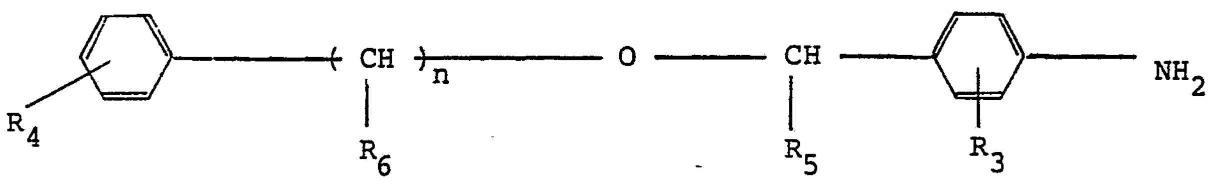

wherein $R_3$, $R_4$, $R_5$, $R_6$ and n have the above-mentioned meanings, with a benzoyl isocyanate of the general formula

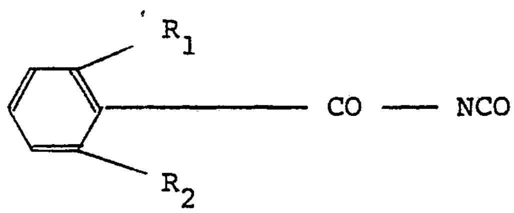

wherein $R_1$ and $R_2$ also have the above-mentioned meanings.

The new compounds can also be prepared by reacting a compound of the general formula

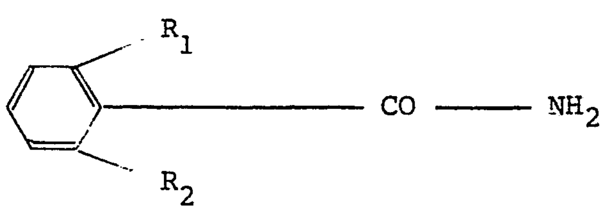

with an isocyanate of the general formula

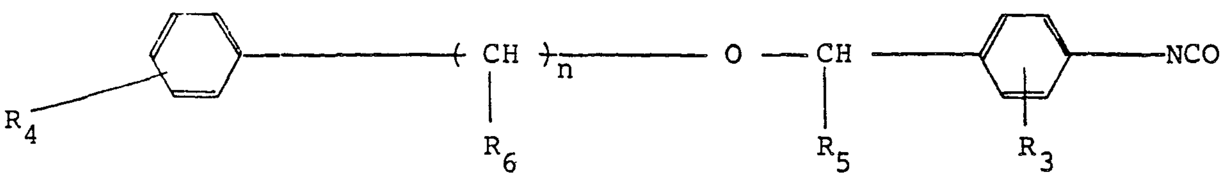

in which formulae the symbols again have the above-mentioned meanings.

The above-mentioned reactions are carried out in the presence of a solvent, for example, an ether, an aromatic hydrocarbon, an alkyl halide or acetonitrile, at a reaction temperature between 0°C and the boiling-point of the solvent used.

Although the above-indicated methods of preparing are the best suitable, the new compounds can also be prepared differently, for example, as described in the above-mentioned Netherlands Patent Application 7105350.

The invention will now be described in greater detail with reference to the following specific examples.

EXAMPLE I

Preparation of N-(2-chlorobenzoyl)-N'-(4-phenoxymethylphenyl) urea (1)

3.63 g of 2-chlorobenzoylisocyanate in 80 ml of dry diethyl ether were added to 4.0 g of 4-phenoxymethylaniline in 80 ml of dry diethyl ether. After stirring for 1 hour at room temperature the formed crystalline precipitate was sucked off, washed successively with diethyl ether, petroleum ether and methanol, and dried. The desired product was obtained in a yield of 5.8 g; melting-point 191-193°C.

The starting aniline was obtained from the corresponding nitro compound by hydrogenation with hydrogen under the influence of Raney nickel as a catalyst in ethanol as a solvent. Phenyl-4-nitrobenzyl ether was prepared from a reaction of 4-nitrobenzyl-bromide and phenol under the influence of anhydrous potassium carbonate in acetone as a solvent.

In a corresponding manner, in which acetonitrile was used as a solvent, if desired, the following compounds

were prepared; the numbers of the compounds correspond to the numbers used before in the specification.

| compound no. | melting-point | compound no. | melting-point |
|---|---|---|---|
| (2) | 179-181°C | (20) | 192-194°C |
| (3) | 192-194°C | (21) | 207-210°C |
| (4) | 199-203°C | (22) | 208-212°C |
| (5) | 203-204°C | (23) | 208-210°C |
| (6) | 187-190°C | (24) | 205-208°C |
| (7) | 193-202°C | (25) | 212-217°C |
| (8) | 189-191,5°C | (26) | 220-223°C |
| (9) | 192-197°C | (27) | 166-168°C |
| (10) | 184-189°C | (28) | 167-169,5°C |
| (11) | 191-194°C | (29) | 187-189°C |
| (12) | 198-203°C | (30) | 160-161°C |
| (13) | 192-194°C | (31) | 170-176°C |
| (14) | 154-156°C | (32) | 177-182°C |
| (15) | 178-180°C | (33) | 138-141°C |
| (16) | 175-178°C | (34) | 141,5-143°C |
| (17) | 165-169°C | (35) | 128-130°C |
| (18) | 172-174°C | (36) | 147-149°C |
| (19) | 157-159°C | (37) | amorphous product |
| | | (38) | 97-108°C |

## EXAMPLE II

The compounds according to the invention are processed to compositions by suspending the compounds in water in the presence of a dispersing agent, for example, lignine sulphonate, and/or a wetting agent, for example, naphthalene sulphonate, an alkyl sulphate, an alkyl benzene sulphonate, an alkyl polyoxyethylene or an alkyl aryl polyoxyethylene.

Young Brussels sprouts plants, approx. 15 cm high, were sprayed with the compositions thus obtained in various concentrations. After drying of the plants, they are placed in plexiglass cylinders and are then infected with 5 larvae of Pieris brassicae (caterpillars of the cabbage white butterfly). The cylinders are then covered with a gauze and stored, a light-dark cycle of 18 hours light and 6 hours dark being used; temperature in

the light 24°C, relative humidity (RV) 70%, temperature in the dark 19°C, 80-90% RV. After 5 days the mortality percentage of the larvae is established. Each experiment has been carried out in triplicate. The results of the experiment are recorded in Table A below. The meanings of the symbols indicated in the Table are as follows:

\+ = 90-100% mortality

± = 50-90% mortality

\- = $<$ 50% mortality

TABLE A

| | Insecticidal activity against larvae of Pieris br. | | | | | | | | |
|---|---|---|---|---|---|---|---|---|---|
| | activity; concentration in mg act. subst. per l. | | | | | | | | |
| compound no. | 300 | 100 | 30 | 10 | 3 | 1 | 0.3 | 0.1 | 0.03 |
| (1) | + | + | + | + | + | + | ± | - | |
| (2) | + | + | + | + | + | + | + | + | ± |
| (3) | + | + | + | + | + | ± | - | | |
| (4) | + | + | + | + | + | - | | | |
| (5) | + | + | + | + | + | + | + | - | |
| (8) | + | + | + | + | + | + | + | - | |
| (9) | + | + | ± | - | | | | | |
| (10) | + | + | + | + | + | + | - | | |
| (11) | + | + | + | + | ± | - | | | |
| (13) | + | + | + | + | + | ± | - | | |
| (14) | + | + | + | + | + | + | + | - | |
| (17) | + | + | + | + | + | + | + | + | - |
| (20) | + | + | + | + | + | + | + | ± | - |
| (23) | + | + | + | + | + | + | ± | - | |
| (24) | + | + | + | + | + | - | | | |
| (26) | + | + | + | + | + | + | - | | |
| (27) | + | + | + | + | + | - | | | |
| (28) | + | + | + | + | + | - | | | |
| (29) | + | + | + | + | ± | - | | | |
| (30) | + | + | + | + | + | + | ± | - | |
| (31) | + | + | + | + | - | | | | |
| (32) | + | + | + | + | - | | | | |
| (33) | + | + | + | + | + | + | ± | - | |
| (34) | + | + | + | ± | - | | | | |
| (36) | + | + | + | + | + | + | ± | - | |
| (37) | + | + | + | + | + | - | | | |
| (38) | + | + | + | + | + | + | ± | | |

EXAMPLE III

20 Larvae of Aedes aegypti (larvae of the yellow fever mosquito) are brought in aqueous suspensions of the active substances obtained according to Example II in various concentrations. The suspensions are kept at a temperature of 25°C for 10 days, during which incubation period the larvae are fed with an aqueous suspension of powdered brown bread and yeast. After 10 days the mortality percentage is determined, taking the natural mortality into account. The results of the experiment are recorded in Table B. The meanings of the symbols are the same as in Example II.

TABLE B

Insecticidal activity against larave of Aedes aegypti

| | activity; concentration in mg act. subst. per l. | | | | | | |
|---|---|---|---|---|---|---|---|
| compound no. | 1 | 0,3 | 0,1 | 0,03 | 0,01 | 0,003 | 0,001 |
| (2) | + | + | + | + | ± | - | |
| (5) | + | + | + | + | ± | - | |
| (8) | + | + | + | + | + | - | |
| (10) | + | + | + | - | | | |
| (13) | + | + | + | + | + | + | ± |
| (14) | + | + | + | + | - | | |
| (17) | + | + | + | + | - | | |
| (20) | + | + | + | + | + | - | |
| (23) | + | + | ± | - | | | |
| (26) | + | + | + | ± | - | | |
| (27) | + | + | + | - | | | |
| (29) | + | + | + | - | | | |
| (30) | + | + | + | + | + | ± | - |
| (31) | + | + | + | + | - | | |
| (32) | + | + | + | + | - | | |
| (33) | ± | ± | - | | | | |
| (36) | + | ± | - | | | | |

EXAMPLE IV

Young potato plants, approx. 15 cm high, are sprayed with the compositions obtained according to Example II in various concentrations. After the plants have dried-up, plexiglass cylinders

are placed over the plants. The plants are then infected with 10 larvae of Leptinotarsa decemlineata (larvae of the Colorado beetle) and stored as indicated in Example II. After 5 days the mortality percentage of the larvae is determined. The results of the experiment are indicated in Table C below. The meanings of the symbols are the same as in Example II.

TABLE C

Insecticidal activity against larvae of Leptinotarsa deceml.

| Compound no. | activity concentration in mg act. subst. per litre | | | | | |
|---|---|---|---|---|---|---|
| | 300 | 100 | 30 | 10 | 3 | 1 |
| (1) | ± | - | | | | |
| (2) | + | + | + | ± | - | |
| (5) | + | + | ± | - | | |
| (8) | + | + | + | - | | |
| (10) | + | + | + | + | - | |
| (13) | + | + | + | + | + | - |
| (14) | + | + | ± | - | | |
| (17) | + | + | + | - | | |
| (20) | + | + | ± | - | | |
| (23) | + | ± | - | | | |
| (28) | + | + | + | ± | - | |
| (30) | + | + | + | + | ± | - |
| (32) | + | + | ± | - | | |
| (33) | + | + | + | + | - | |
| (36) | + | ± | - | | | |
| (37) | + | ± | - | | | |
| (38) | + | + | + | - | | |

CLAIMS:

1. Compounds of the general formula

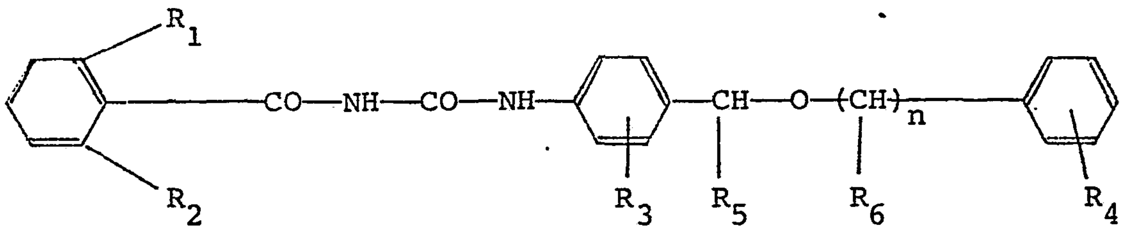

wherein $R_1$ is a hydrogen atom, a chlorine atom, a fluorine atom or a methyl group,

$R_2$ is a chlorine atom, a fluorine atom, or a methyl group,

$R_3$ is a hydrogen atom or one or two substituents selected from the group consisting of a halogen atom and an alkyl group having 1 to 4 carbon atoms,

$R_4$ is a hydrogen atom or from one to three substituents selected from the group consisting of a halogen atom and an alkyl group, alkoxy group or alkylthio group having 1 to 4 carbon atoms and substituted or not substituted with halogen,

$R_5$ and $R_6$ are equal or different and represent a hydrogen atom or an alkyl group having 1 to 4 carbon atoms and substituted or not substituted with halogen, and

$\underline{n}$ is 0 or 1.

2. Compounds of the general formula

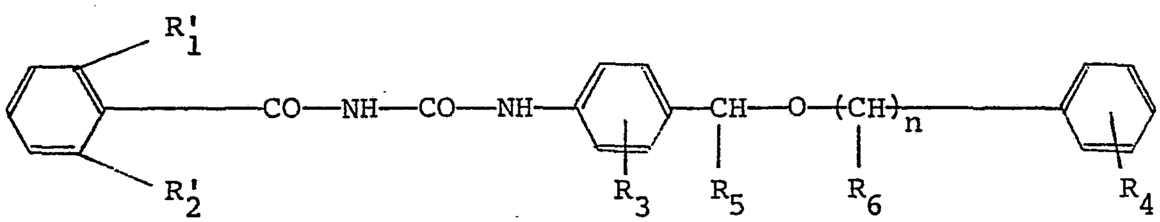

wherein either $R_1'$ and $R_2'$ both are fluorine atoms,

or $R_1'$ is a hydrogen atom and $R_2'$ is a chlorine atom,

and $R_3$, $R_4$, $R_5$, $R_6$ and $\underline{n}$ have the meanings given in Claim 1.

3. N-(2,6-difluorobenzoyl)-N'-[4-(2,4-dichlorophenoxymethyl)phenyl]urea.

4. A method of preparing compounds of the general formula

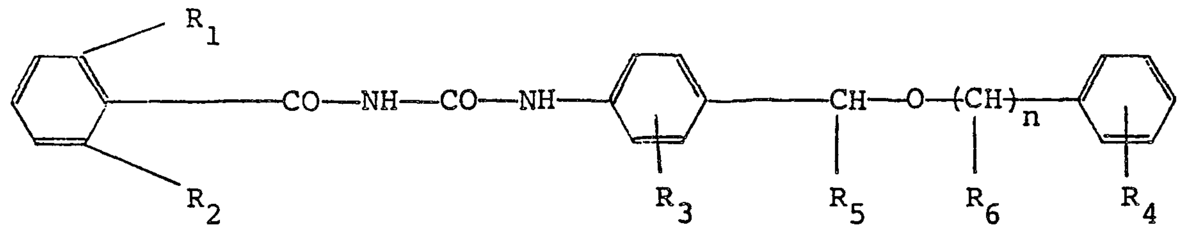

wherein $R_1$, $R_2$, $R_3$, $R_4$, $R_5$, $R_6$ and n have the meanings given in Claim 1, characterized in that the compounds are prepared by reacting a substituted aniline of the general formula

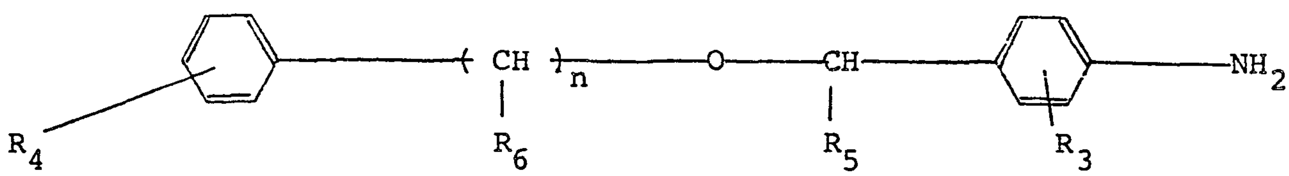

wherein $R_3$, $R_4$, $R_5$, $R_6$ and n have the meanings stated in Claim 1, with a benzoylisocyanate of the general formula

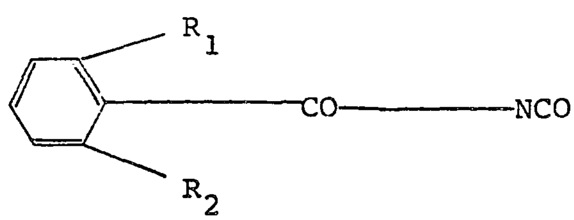

wherein $R_1$ and $R_2$ have the meanings also stated in Claim 1.

5. A method of preparing compounds of the general formula

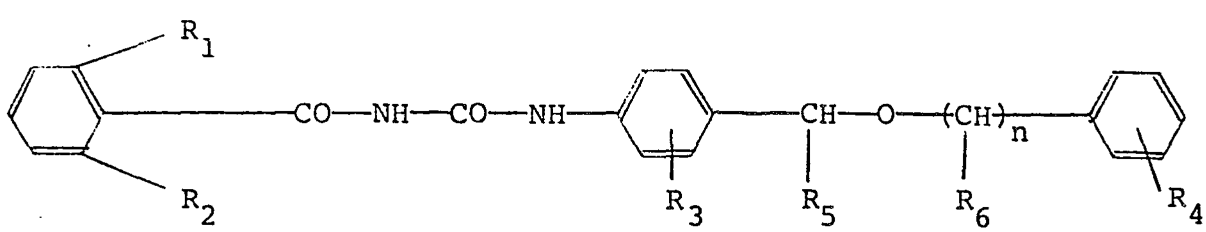

wherein $R_1$, $R_2$, $R_3$, $R_4$, $R_5$, $R_6$ and n have the meanings given in Claim 1, characterized in that the compounds are prepared by reacting a compound of the general formula

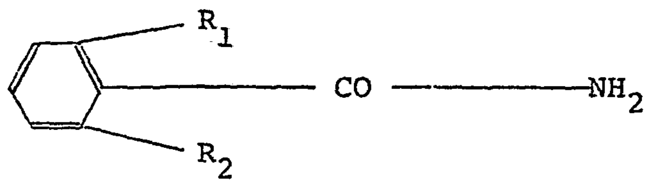

with an isocyanate of the general formula

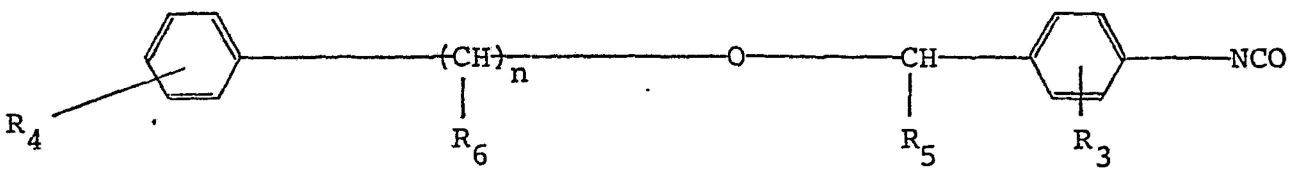

in which formulae the symbols have the meanings stated in Claim 1.

6. Insecticidal composition, characterized in that, in addition to a liquid of solid carrier material, the composition comprises a compound of the general formula

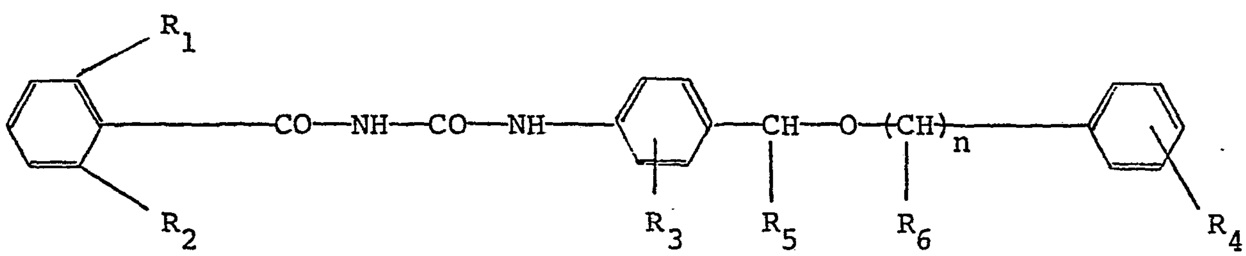

wherein $R_1$, $R_2$, $R_3$, $R_4$, $R_5$, $R_6$ and n have the meanings given in Claim 1.

7. A composition as claimed in Claim 6, characterized in that the active constituent is a compound of the general formula

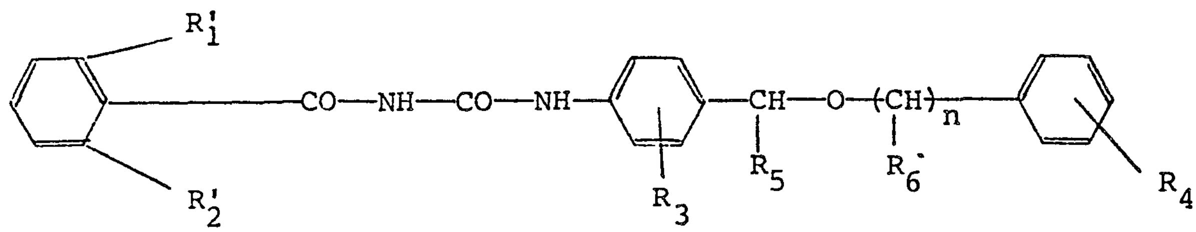

wherein $R_3$, $R_4$, $R_5$, $R_6$ and n have the meanings given in Claim 1 and $R_1'$ and $R_2'$ have the meanings given in Claim 2.

8. A composition as claimed in Claim 6, characterized in that the active constituent is N-(2,6-difluorobenzoyl)-N'-[4-(2,4-dichlorophenoxymethyl)phenyl]urea.

9. A method of preparing an insecticidal composition, characterized in that a compound of the general formula

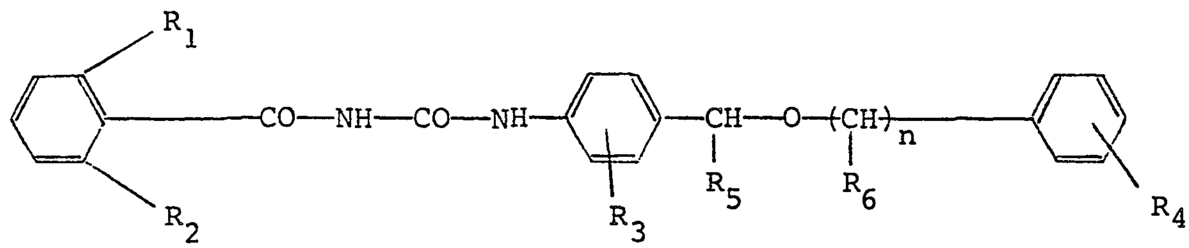

wherein $R_1$, $R_2$, $R_3$, $R_4$, $R_5$, $R_6$ and n have the meanings given in Claim 1,
is mixed with a liquid or solid inert carrier material, if desired with the addition of other pesticidal compounds, artificial manures, and/or auxiliary substances, for example, wetting agents, emulsifiers, dispersing agents and stabilizers.

10. A method of controlling insects, characterized in that the infected area is treated with a composition as claimed in any of the preceding Claims 6-8 in a dosage from 10 to 5,000 g of active substance per hectare.